# EUROPEAN PATENT SPECIFICATION

(11) **EP 4 121 174 B1**
(45) Date of publication and mention of the grant of the patent: **09.09.2026**
(21) Application number: 21711853.8
(22) Date of filing: 11.03.2021
(51) Int. Cl.: A61Q 5/02, A61Q 5/12, A61Q 19/10, A61K 8/41, A61K 8/44, A61K 8/49, A61K 8/60, A61K 8/65

(54) **PERSONAL CARE COMPOSITION, METHOD FOR USING SUCH COMPOSITIONS AND ITS IMPROVEMENT OF DEPOSITION EFFECT**
PERSÖNLICHE PFLEGEZUSAMMENSETZUNG, VERFAHREN ZUM VERWENDEN SOLCHER ZUSAMMENSETZUNGEN UND IHRE VERBESSERUNG DES ABLAGERUNGSEFFEKTS
COMPOSITION DE SOINS PERSONNELS, PROCÉDÉ D'UTILISATION DE TELLES COMPOSITIONS ET SON AMÉLIORATION DE L'EFFET DE DÉPÔT

(30) Priority: 18.03.2020 WO PCT/CN2020/080027
(43) Date of publication of application: 25.01.2023
(73) Proprietor: BASF SE, 67056 Ludwigshafen am Rhein (DE)
(72) Inventor: ZHANG, Zhen, Shanghai, Shanghai 200137 (CN); ZHU, Ben Chuan, Shanghai, Shanghai 200137 (CN)
(74) Representative: BASF IP Association
(86) International application number: PCT/EP2021/056259
(87) International publication number: WO 2021/185675

(56) References cited:
- EP-A1- 2 786 742
- EP-A1- 3 290 500
- WO-A1-2020/178048
- WO-A1-2020/262567
- FR-A1- 2 855 752
- GB-A- 2 533 975
- US-A1- 2014 349 902
- US-A1- 2017 071 842
- US-A1- 2019 307 657
- DATABASE GNPD [online] MINTEL; 27 October 2021 (2021-10-27), ANONYMOUS: "Sea Salt Scalp Scrub", XP093255150, retrieved from https://www.gnpd.com/sinatra/recordpage/9103612/ Database accession no. 9103612
- DATABASE GNPD [online] MINTEL; 19 January 2017 (2017-01-19), ANONYMOUS: "*Energy Activate Cream", XP055812357, retrieved from https://www.gnpd.com/sinatra/recordpage/4568215/ Database accession no. 4568215
- DATABASE GNPD [online] MINTEL; 19 December 2016 (2016-12-19), ANONYMOUS: "Nourishing Lip Care SPF 25 PA+++", XP055812369, retrieved from https://www.gnpd.com/sinatra/recordpage/4488083/ Database accession no. 4488083
- ANONYMOUS: "Toning lotion", GNPD, MINTEL, 1 December 2016 (2016-12-01), XP002772933
- LG HOUSEHOLD: "Essential Skin Toner", GNPD, MINTEL, 1 January 2015 (2015-01-01), XP002792259

## Description

### Technical Field

The present invention relates to a personal care composition, a method for using such composition and a method of facilitating or enhancing deposition effect when the composition being applied onto keratinous materials.

### Background Art

Many personal care or cosmetic compositions comprise active substances which are applied to the skin and/or hair in a rinse-off formulation. Whereas the rinse-off formulation is rinsed off again from the skin and/or hair following use, the active substances should be deposited on the skin and/or hair. The active substances are usually mixed directly into the compositions, for example shampoos, shower gels, face cleansers or solid or liquid soaps. This procedure has the disadvantage that in most cases upon use only small amount of the active substances remain on the skin, which can develop their effect there. The majority of the active substances is usually washed off again with the rinse-off formulation. This leads to large amounts of costly active substances having to be incorporated into the formulations so as to achieve a desired effect. However, when suitably high amounts of active substances are used in the rinse-off formulations, an undesired skin irritation is often observed when the formulations are used. Here, active ingredients are in particular cationic active ingredients which are often used in personal care formulation, for instance hexamidine diisethionate, which is a novel antidandruff agent used in hair cosmetic formulation; another example is alkaloid quinine or its salts, which is also claimed for scalp refreshing and preventing hair loss. However, it would be more challenging to retain such kind of active ingredients which is hydrophile or even with relative higher water solubility in the formulation and to effectively develop their functional benefits onto skin and/or hair.

Many attempts have been made for improved deposition effect by developing coacervation technology, in particular in rinse-off products. However, the technology is well developed more for hydrophobic active ingredients. In recent times there is an increasing demand for personal care compositions, in particular for rinse-off personal care composition, on the one hand, allow the amount of active ingredients used to be kept low to avoid skin irritation and thus to reduce costs, and on the other hand, allow an improved deposition effect of active ingredients which are hydrophile or even with relative good water solubility and bring about desirable benefits during use.

It is thus an object of the present invention to address the ever increasing demand in the market for cosmetic cleansing and conditioning compositions which provide an improved deposition of active ingredients, in particular an improved deposition of hydrophile cationic or pseudo-cationic active ingredient, and also provide a good cleansing and conditioning effects onto skin and/or hair.

Sophorolipids are all-natural and consumer-friendly fermentation surfactant which are produced by several yeast species, e.g. Starmerella. The hydrophilic moiety of the biosurfactant molecule is a disaccharide (i.e., sophorose), and the hydrophobic portion is an omega- or (omega-1)-hydroxy fatty acid attached to the sophorose via a glycosidic bond. The fatty acid chain, most commonly containing 16- and 18-carbon atoms, may be unsaturated and lactonized to the disaccharide. Sophorolipids, as biosurfactants, can serve as environmentally friendly surfactants being used widely in detergent and cosmetic formulations. EP 0550276 A1 describes a personal care composition comprising a mild and foaming glycolipid used with a co-surfactant (e.g. SLS). WO 1998016192 A1 discloses a germicidal composition suitable for cleaning fruits, vegetables, skin and hair. The composition may comprise a mixture of anionic surfactant and sophorolipids biosurfactant. US patent application 2014/0349902 A1 discloses a hair and skin cleansing compositions comprising water, at least one biosurfactant and at least one fatty acid for obtaining an improved skin care benefit and foaming properties.

DATABASE GNPD [Online], MINTEL; 19 January 2017 (2017-01-19), anonymous: "*Energy Activate Cream*", XP055812357, Database accession no. 4568215 discloses the combination of a glycolipid with protein hydrolysates (hydrolysed collage and hydrolysed elastin) or basic amino acids (histidine, arginine, and lysine HCI).

DATABASE GNPD [Online], MINTEL; 19 December 2016 (2016-12-19), anonymous: "Nourishing Lip Care SPF 25 PA+++", XP055812369, Database accession no. 4488083 discloses the combination of a glycolipid (mannosylerythritol lipid) with hydrogenated lecithin and phospholipid.

ANONYMOUS: "Toning lotion", GNPD, MINTEL, 1 December 2016 (2016-12-01), XP002772933 discloses the combination of a glycolipid (mannosylerythritol lipid) with caffeine (i.e. alkaloid compound), lecithin, hydrogenated lecithin and phospholipids.

LG HOUSEHOLD: "Essential Skin Toner", GNPD, MINTEL, 1 January 2015 (2015-01-01), XP002792259 discloses the combination of glycolipid (mannosylerythritol lipid) with protein hydrolysates (hydrolysed collage and hydrolysed elastin).

US 2014/349902 A1 discloses compositions comprising water, at least one biosurfactant and at least one fatty acid, which are characterized in that the fraction of the sum of all surfactants in the composition is from 1 to 30% by weight, and that the fraction of fatty acid, based on the sum of fatty acid and surfactants, is from 0.1 to 20% by weight, and to the use thereof as or for producing bath additives, shower gel, shampoos, conditioners, body cleansers or skin cleansers.

EP 3 290 500 A1 discloses agents with polyoxyalkylene carboxylate and glycolipid comprising rhamnolipid, sophorolipid, cellobioselipid, trehaloselipid, mannosylerythritol lipid, for use as washing, care and cleaning agents.

US 2017/071842 A1 discloses cosmetic cleansing agents including biosurfactants and having prebiotic activity.

FR 2 855 752 A1 discloses cosmetic uses of sophorolipids, as slimming agents and/or as active agents stimulating the synthesis of leptin by adipocytes, for the manufacture of a cosmetic composition intended to reduce subcutaneous excess fat.

GB 2 533 975 A discloses an antibiofilm composition comprising at least one quorum sensing inhibitor, at least one iron-chelating agent and at least one antimicrobial agent.

US 2019/307657 A1 discloses oral care compositions comprising at least one biosurfactant and fluoride.

EP 3 989 923 A1 discloses a composition comprising (a) at least one biosurfactant; and (b) at least one carboxybetaine polymer.

EP 3 934 613 A1 discloses the use of rhamolipids for the deposition of at least one substance from a medium on a surface.

The present application has now discovered unexpectedly that a personal care composition containing a glycolipid biosurfactant and a hydrophile cationic or pseudo-cationic active ingredient makes it possible to enhance and/or facilitate a deposition effect of the hydrophile cationic or pseudo-cationic active ingredient onto keratinous materials, more particularly, the personal care composition is a rinse-off composition.

### Summary of Invention

The subject of the present invention is thus a personal care composition comprising at least:
a) a glycolipid biosurfactant,
b) a hydrophile cationic or pseudo-cationic active compound;
wherein the glycolipid is sophorolipid, and wherein the hydrophile cationic or pseudo-cationic active compound comprises one or more amidine compounds or salts thereof and/or one or more alkaloid compounds selected from the group consisting of quinine and its salts.

Preferably, 10 to 90 wt.% of the sophorolipid biosurfactant is in the acidic form.

In a further aspect, the present invention provides personal care compositions and methods for enhancing or facilitating a deposition of hydrophile cationic or pseudo-cationic active compound onto keratinous materials, comprising at least one step of applying to the keratinous materials an effective amount of the personal care composition, in particular, during or after rinse application.

In another aspect, the present invention is also directed to use of the present personal care composition for enhancing or facilitating a deposition of hydrophile cationic or pseudo-cationic active compound onto keratinous materials. In still another aspect, the present invention is directed to use of such personal care composition for simultaneously caring for and washing keratinous materials, such as hair and/or skin.

It was surprisingly and unexpectedly found that the above-described objective be achieved by the personal care composition containing a glycolipid biosurfactant and a hydrophile cationic or pseudo-cationic active ingredient. The composition brings about an enhanced deposition effect of the hydrophile cationic or pseudo-cationic active ingredients onto keratinous materials, in particular, the personal care composition is a rinse-off product.

### Detailed Description of The Invention

Throughout the description, including the claims, the term "comprising one" or "comprising a" should be understood as being synonymous with the term "comprising at least one", unless otherwise specified, and "between" should be understood as being inclusive of the limits.

The terms "a", "an" and "the" are used to refer to one or to more than one (i.e., to at least one) of the grammatical object of the article.

The term "and/or" includes the meanings "and", "or" and also all the other possible combinations of the elements connected to this term.

The term "hydrophilic" used herein, means that the compound has a strong affinity to water.

The term "charge density" as used herein, means the ratio of the number of positive charges on a monomeric unit (of which a polymer is comprised) to the molecular weight of said monomeric unit. The charge density multiplied by the polymer molecular weight determines the number of positively charged sites on a given polymer chain.

The term "active compound" used herein, refers to the ingredient or ingredients that improve or maintain the health of the dermal barrier.

The term "pseudo-cationic compound" used herein, refers to compounds that do not possesses an inherent positive charge, but do possess behavior similar to positively charged compounds.

The pseudo-charged behavior arises in these compounds due to electro donating or electro receiving atoms and/or groups within the compounds.

The term "cosmetically acceptable" as used herein, means that the compositions, formulations, or components described are suitable for use in contact with human keratinous materials without undue toxicity incompatibility instability allergic response.

The term "keratinous material" used herein, means to comprise hair, lips, skin, scalp and superficial body growths such as eyelashes, eyebrows and nails.

The term "rinse-off" used herein, refers to the composition are used in a context whereby the composition is ultimately rinsed or washed from the treated surface, (e.g. skin or hair) either after or during the application of the product.

It should be noted that in specifying any range of concentration, weight ratio or amount, any particular upper concentration, weight ratio or amount can be associated with any particular lower concentration, weight ratio or amount, respectively. Unless otherwise specified, all percentages are by weight.

The present invention is directed to a personal care composition comprising at least:
a) a glycolipid biosurfactant,
b) a hydrophile cationic or pseudo-cationic active compound;
wherein the glycolipid is sophorolipid, and wherein the hydrophile cationic or pseudo-cationic active compound comprises one or more amidine compounds or salts thereof and/or one or more alkaloid compounds selected from the group consisting of quinine and its salts.

The personal care composition of the present invention can enhance and/or facilitate a noteworthy deposition of hydrophile cationic or pseudo-cationic active compound onto keratinous materials, in particular the hair, comprising at least one step of applying to the keratinous materials an effective amount of the composition of the present invention.

The composition of the invention is a personal care composition or a cosmetic composition, preferably a personal care cleansing composition, that is to say a composition used for the purpose of cleansing, conditioning, grooming, beautifying, or otherwise enhancing the appearance of the human body. Personal care products include skin care products, cosmetic products, antiperspirants, deodorants, perfume, toiletries, soaps, bath oils, feminine care products, hair-care products, oral hygiene products, depilatories, including shampoos, conditioners, hair straightening products and other haircare products, color cosmetics such as lipstick, creams, make-up, skin creams, lotions (preferably comprised of water-in-oil or oil-in- water emulsions), shave creams and gels, after-shave lotions and shave-conditioning compositions and sunscreen products, among numerous others. In preferred aspects, personal care compositions according to the present invention include haircare and skin care products, especially shampoos, conditioners, rinses, detangler products, hair color products, body washes, make-up, lipstick, skin creams and other skin-care products.

In some embodiments, the personal care composition of the present invention can be used in a traditional Chinese medicine composition which comprises Chinese herbal medicine and is prepared based on traditional Chinese medicine theory. Some examples of Chinese herbal medicines include, for example ginseng which can nourish hair growths and strength, sophora like sophora flavescens extract which is helpful for hair growths and prevents hair loss, vaccaria for blood circulation and mulberry for desirable moistening effect.

### Glycolipid biosurfactant

The composition of the present invention comprises at least one glycolipid biosurfactant, wherein the glycolipid is sophorolipid.

Biosurfactant is understood to be substances that are formed by microorganisms and are often expelled from the cell. Like classic surfactants, biosurfactant is surface-active substance that reduce the surface tension of liquids and thereby promote the mixing of aqueous (hydrophilic) and water-repellent (hydrophobic) phases. Biosurfactant can be produced under gentle production conditions that require little energy. They are generally easily biodegradable and are very environmentally friendly. Moreover, they are not toxic, nor are any toxic byproducts produced during the production thereof. Carbohydrate, in particular sugar, e.g. glucose, and/or lipophobic carbon sources such as fats, oils, partial glycerides, fatty acids, fatty alcohols, long-chain saturated or unsaturated hydrocarbons, are used as raw materials for the microbial production of said biosurfactant. According to the present invention, the biosurfactant is preferably biosurfactant produced by fermentation.

Sophorolipids are produced by fermentation using yeasts such as *Starmerella* (*Candida*) *Bombicola* (also known as *Torulopsis bombicola*), *Yarrowia lipolytica, Candida apicola* (*Torulopsis apicola*) and *Candida bogoriensis,* by growing said yeasts on sugars, hydrocarbons, plant oils or mixture thereof. Sophorolipids have the following formulae (a) (lactone form) and (b) (free acid), the two forms typically being provided in a mixture,

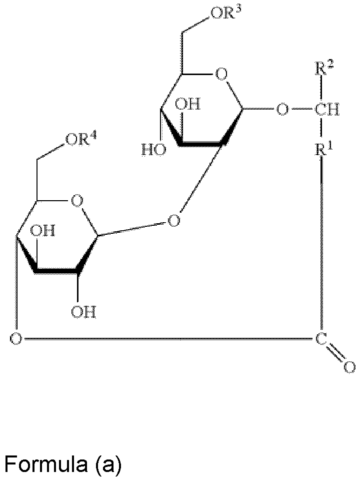

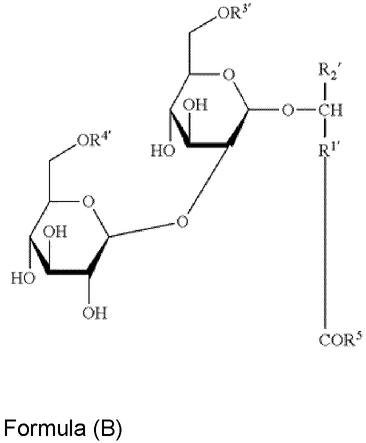

Where R¹ and R^{1'} independently represent saturated hydrocarbon chains or single or multiple, in particular single, unsaturated hydrocarbon chains having 8 to 20, in particular 12 to 18, carbon atoms, more preferably 14 to 18 carbon atoms, which can be linear or branched and can include one or more hydroxy groups, R² and R^{2'} independently represent a hydrogen atom or an methyl group. Sophorolipid in which R¹ and R^{1'} are single, unsaturated, linear hydrocarbon chains having 15 carbon atoms are preferred. It is also preferred for R² and R^{2'} to represent a methyl group or a hydrogen atom or a saturated alkyl functional group or a single or multiple, in particular single, unsaturated alkyl functional group having 1 to 9 carbon atoms, which can be linear or branched and can include one or more hydroxy groups, and R³, R^{3'} , R⁴ and R^{4'} independently represent a hydrogen atom or an acetyl group. R⁵ represents a hydroxy group.

According to the present invention, sophorolipids in which the acidic form and the lactone form in a mixture are preferred, preferably about 10 to about 90 wt.%, more preferably about 20 to about 60 wt.%, still more preferably about 25 wt.% to 40 wt.% of the sophorolipid being in acidic form and the remainder of the sophorolipid being in the lactone form.

Sophorolipids being suitable used for the present invention can be obtained commercially, for example under the name Sopholiance S from Soliance, and under the name ACS-Sophor^{®} from Allied carbon solution. The sophorolipid supplied by Allied Carbon Solution, about 30 wt.% is present in the free acid form, in a mixture with the lactone form.

According to any one of the invention embodiments, the composition of the invention comprises from 0.1 % to 30 wt. % of sophorolipid surfactant relative to the total weight of the composition, preferably from 0.2 % to 20 wt.%, more particularly from 0.2 % to 15 wt.%, still more preferably from 0.5 % to 10 wt.%.

### Hydrophile cationic or pseudo-cationic active compound

The composition of the present invention comprises at least one hydrophile cationic or pseudo-cationic active compound, wherein the hydrophile cationic or pseudo-cationic active compound comprises one or more amidine compounds or salts thereof and/or one or more alkaloid compounds selected from the group consisting of quinine and its salts.

In some embodiments, the hydrophile cationic or pseudo-cationic active compounds include one or more amidine compounds, such as hexamidine, pentaamine, benzamidine, their salts, and/or their mixtures. Amidine compounds exhibits a broad antimicrobial spectrum of activity against bacteria, yeast and fungi. Those compounds are useful in the composition of the present invention as hair growth regulator, namely mammalian hair growth, including reducing, modulating, inhibiting, attenuating, retarding, promoting, enhancing, and/or the diminution of hair growth. "Mammalian hair," as referenced herein, includes hair on any part of the body of a mammal and may include facial, cranial, or body hair. Non-limiting examples of amidine hair growth regulators useful in the present invention include:
- Hexamidine 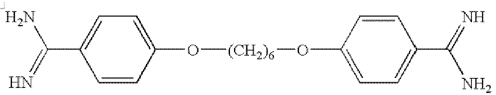
- Pentamidine 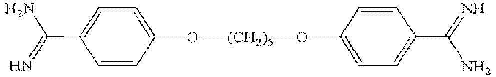
- Benzamidine

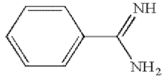

The composition of the present invention preferably comprises from about 0.0001% to about 10% of the amidine compound, more preferably from about 0.001% to 5% and more preferably still from about 0.01% to 1%, and most preferably from about 0.02% to 0.5% by weight of the composition.

In some preferred embodiments, the composition of the present invention comprises a safe and effective amount of one or more of hexamidine compounds, its salts, and isomers and tautomers, including but not being limited to organic acids and mineral acids, for example sulfonic acid, carboxylic acid.

More preferably still, the hexamidine is hexamidine diisethionate, commercially available as Elastab^{®} HP100 from BASF.

In some embodiments, the compositions of the present invention further comprise one or more basic amino acids as hydrophile cationic or pseudo-cationic active compounds which are known to be important for the nourishment of keratinous materials, like nourishment of hair root and the growth of human hair. By "basic amino acid" is meant the naturally occurring basic amino acids, such as arginine, lysine and histidine, as well as any basic amino acid having a carboxyl group and an amino group in the molecule, which is water-soluble and provides an aqueous solution with a pH of 7 or above. Accordingly, basic amino acids include, but are not limited to, arginine, lysine, citrulline, ornithine, creatine, histidine, diaminobutanoic acid, diaminoproprionic acid, salts thereof or combination thereof. In a preferred embodiment, the basic amino acid is creatine, diaminobutanioic acid or diaminoproprionic acid, salts thereof or combination thereof. The composition of the present invention comprises basic amino acid compound in free or cosmetically acceptable salt form. By "cosmetically acceptable salt form" is meant a salt form which is to keratinous materials such as hair or skin in the concentration provided. The composition of the present invention preferably comprises from about 0.001% to about 20% of the basic amino acids, more preferably from about 0.01% to 10% and more preferably still from about 0.1% to 5% by weight of the composition.

In some embodiments, the composition of the present invention further comprises hydrophile cationic or pseudo-cationic active compounds which can be one or more imidazole antimicrobials, such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole, itraconazole or combination thereof. As antimicrobial active, a safe and effective amount of an antimicrobial active can be added to the present compositions. In some embodiments, the composition may comprise from about 0.0001%, 0.001%, 0.01%, 0.05%, 0.1%, 0.5% or 1% to about 30%, 25%, 20%, 10%, 7%, 5% or 3% by weight of the composition, of one or more imidazoles antimicrobials.

In some embodiments, the composition of the present invention comprises one or more alkaloid compounds selected from the group consisting of quinine and its salts (bisulfate, sulfate, chloride, oleate, and tannate), which can be used in the present invention for minimizing hair loss and stimulating hair growth. The composition of the present invention preferably comprises from about 0.001% to about 20% of the alkaloid compound, more preferably from about 0.01% to 10% and more preferably still from about 0.1% to 5% by weight of the composition.

In some embodiments, the composition of the present invention further comprises hydrophile cationic or pseudo-cationic active compounds which can be biguandine compounds. By "biguandine compound" is meant a chemical having the following functional group: 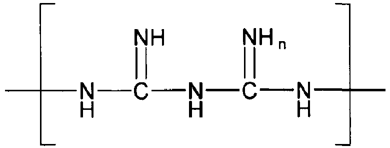 wherein n=1 or 2. When n=2, the nitrogen atom of the =NHₙ group may be drawn as tetravalent and hence positively charged.

Preferably, a biguanide compound is used in the present invention as antimicrobial agent, such as a polymeric biguanide compound. A particularly preferred polymeric biguanide compound is polyhexamethylenebiguanide (PHMB).

In one embodiment, the composition of the present invention may comprise o-tolyl biguandine, which is used in personal care composition as antioxidant agent, in particular in skin care composition. The composition of the present invention preferably comprises from about 0.0001% to about 10% of the biguandine compound, more preferably from about 0.001% to 5%, more preferably still from about 0.01% to 1%, and most preferably 0.02% to 0.5% by weight of the composition.

In some embodiments, the composition of the present invention further comprises one or more cationic proteins and/or protein hydrolysates as hydrophile cationic or pseudo-cationic active compounds. Suitable protein hydrolysates are product mixtures which can be obtained by acid, alkaline or enzymatically catalyzed degradation of proteins. The proteins suitable for the present invention come from animal, vegetables and/or marines. Suitable animal protein hydrolysates are, for example, elastin, collagen, keratin, silk and/or milk protein hydrolysates, which may also be present in the form of salts. Such products are, for example, under the trademarks Dehylan^{®} (BASF SE), Collapuron^{®} (BASF SE), Nutrilan^{®} (BASF SE), and kerasol tm^{®} (Croda) sold. Suitable protein hydrolysates of plant origin are, for example, soybean, almond, rice, pea, potato, rapeseed and/or wheat protein hydrolysates. Such products are, for example, under the trademarks Gluadin^{®} (BASF SE) and Crotein^{®} (Croda) available. Suitable protein hydrolysates of marine origin include, for example, collagen hydrolysates of fish or algae, and protein hydrolysates of mussels or pearl hydrolysates. It is also possible to use cationized protein hydrolysates, it being possible for the underlying protein hydrolysate to be derived from the animal, vegetable and / or marine sources described above. The protein hydrolysates on which the cationic derivatives are based can be obtained from the corresponding proteins by chemical, in particular alkaline or acid hydrolysis, by enzymatic hydrolysis and/or a combination of both types of hydrolysis. The hydrolysis of proteins usually results in a protein hydrolysate having a molecular weight distribution of about 100 to 50,000 daltons. Preference is given to those cationic protein hydrolyzates has a molecular weight 200 to 25,000 daltons, for example 200 to 10000 daltons, for example 250 to 5000 daltons. Furthermore, cationic protein hydrolysates are to be understood as meaning quaternized amino acids and mixtures thereof. The quaternization of the protein hydrolysates or amino acids is often carried out using quaternary ammonium salts such as N,N-dimethyl-N-(n-alkyl) -N-(2-hydroxy-3-chloro-n-propyl) ammonium halides. In addition, the cationic protein hydrolysates may also be further derivatized. Typical examples of the cationic protein hydrolysates and derivatives include the products known under the INCI names: cocodimonium hydroxypropyl hydrolyzed collagen, cocodimopnium hydroxypropyl hydrolyzed casein, cocodimonium hydroxypropyl hydrolyzed collagen, cocodimonium hydroxypropyl hydrolyzed keratin , cocodimonium hydroxypropyl hydrolyzed rice protein, cocodimonium hydroxypropyl hydrolyzed silk, cocodimonium hydroxypropyl hydrolyzed soy protein, cocodimonium hydroxypropyl hydrolyzed wheat protein, cocodimonium hydroxypropyl silk amino acids, hydroxypropyl arginine lauryl/myristyl ether HCI, hydroxypropyltrimonium gelatin, hydroxypropyltrimonium hydrolyzed casein, hydroxypropyltrimonium hydrolyzed collagen, hydroxypropyltrimonium hydrolyzed conchiolin protein, hydroxypropyltrimonium hydrolyzed keratin, hydroxypropyltrimonium hydrolyzed rice bran protein, hydroxyproypltrimonium hydrolyzed silk, hydroxypropyltrimonium hydrolyzed soy protein, Hydroxypropyl Hydrolyzed vegetable protein, hydroxypropyltrimonium hydrolyzed wheat protein, hydroxypropyltrimonium hydrolyzed wheat protein/siloxysilicate, lauridimonium hydroxypropyl hydrolyzed soy Protein, lauridimonium hydroxypropyl hydrolyzed wheat protein, laurodimonium hydroxypropyl hydrolyzed wheat protein/siloxysilicate, lauryldimonium hydroxypropyl hydrolyzed casein, lauryldimony hydroxypropyl hydrolyzed collagen, lauryldimonium hydroxypropyl hydrolyzed keratin, lauryldimonium hydroxypropyl hydrolyzed silk, lauryldimonium hydroxypropyl hydrolyzed soy protein, steardimonium hydroxypropyl hydrolyzed casein, steardimonium hydroxypropyl hydrolyzed collagen, steardimonium hydroxypropyl hydrolyzed keratin, steardimonium hydroxypropyl hydrolyzed rice protein, steardimonium hydroxypropyl hydrolyzed silk, steardimonium hydroxypropyl hydrolyzed soy protein, steardimonium hydroxypropyl hydrolyzed vegetable protein, steardime hydroxy hydroxy hydrolyzed wheat protein, steartrimonium hydroxyethyl hydrolyzed collagen, quaternium-76 hydrolyzed collagen, quaternium-79 hydrolyzed collagen, quaternium-79 hydrolyzed keratin, quaternium-79 hydrolyzed milk protein, quaternium-79 hydrolyzed silk, quaternium-79 hydrolyzed soy protein, quaternium-79 hydrolyzed wheat protein. According to any one of the invention embodiments, the composition contains from 0.01 to 30 wt.% of at least one cationic protein and/or protein hydrolysates, preferably from 0.1 to 20 wt.%, more preferably from 0.5 to 10 wt.%, and most preferably from 1 to 5 wt.% based on the total weight of the composition.

### Conditioning agent

According to any one of the invention embodiments, the composition of the invention may further comprise a conditioning agent, especially a cationic or ampholytic conditioning agent. Suitable cationic conditioning polymers that can be used include cationic polysaccharide polymers, such as cationic cellulose derivatives, cationic starch derivatives, and cationic guar gum derivatives. Suitably, such cationic polysaccharide polymers have a charge density in the range from 0.1 to 4 meq/g. In some embodiments, the conditioning agent may be a cationic cellulose. Cationic cellulose is available from Amerchol Corp. (Edison, N.J., USA) in their Polymer JR (trademark) and LR (trademark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, N.J., USA) under the tradename Polymer LM-200.

According to any one of the invention embodiments, one of suitable cationic conditioning polymers can be cationic guar polymer, which is cationally substituted galactomannan (guar) gum derivatives. Guar gum for use in preparing these guar gum derivatives is typically obtained as a naturally occurring material from the seeds of the guar plant. The guar molecule itself is a straight chain mannan, which is branched at regular intervals with single membered galactose units on alternative mannose units. The mannose units are linked to each other by means of □(1-4) glycosidic linkages. The galactose branching arises by way of an □(1-6) linkage. Cationic derivatives of the guar gums are obtained by reaction between the hydroxyl groups of the polygalactomannan and reactive quaternary ammonium compounds. The cationic guar polymer has a weight average molecular weight of 10,000 to 5,000,000 g/mol, and has a charge density of from about 0.05 to about 2.5 meq/g; preferably, the cationic guar polymer has a weight average molecular weight of 15,000 to 2,500,000 g/mol, more preferably, of 200,000 to 1,500,000 g/mol; and has a charge density of from 0.2 to 2.2 meq/g, or 0.3 to 2.0 meq/g, or from 0.4 to 1.8 meq/g. Suitable cationic guar polymers include cationic guar gum derivatives, such as guar hydroxypropyltrimonium chloride. In some embodiments, the cationic guar polymer is a guar hydroxypropyltrimonium chloride. Specific examples of guar hydroxypropyltrimonium chlorides include the Jaguar^{®} series commercially available from Rhone-Poulenc Incorporated. Other suitable guar hydroxypropyltrimonium chloride are: Hi-Care 1000, which has a charge density of about 0.7 meq/g and a molecular weight of 600,000 g/mol and is available from Rhodia, N-Hance 3269 and N-Hance 3270, which has a charge density of about 0.7 meq/g and a molecular weight of about 425,000 g/mol and is available from ASI. AquaCat CG518 has a charge density of 0.9 meq/g and a molecular weight of 50,000 g/mol and is available from ASI.

According to any one of the invention embodiments, one of suitable cationic conditioning polymer can be water-soluble cationically modified starch polymers. As used herein, the term "cationically modified starch" refers to a starch to which a cationic group is added prior to degradation of the starch to a smaller molecular weight, or wherein a cationic group is added after modification of the starch to achieve a desired molecular weight. The definition of the term "cationically modified starch" also includes amphoterically modified starch. The term "amphoterically modified starch" refers to a starch hydrolysate to which a cationic group and an anionic group are added. The cationically modified starch polymers for use in the personal washing compositions of the present invention have a molecular weight from about 850,000 to about 15,000,000 and/or from about 900,000 to about 5,000,000. The cationically modified starch polymers used in the present invention have a charge density of from 0.2 about meq/g to about 5 meq/g, preferably from about 0.2 meq/g to about 2 meq/g.

Other cationic or ampholytic conditioning agent known in the art may be used provided that they are compatible with the inventive composition. Mention may be made especially of synthetic cationic polymers (for example polymers comprising units having a quaternary ammonium group or a tertiary ammonium group, and optionally neutral units) and of synthetic ampholytic copolymers (for example polymers comprising units having a quaternary ammonium group or a tertiary ammonium group, units having an anionic (usually acidic) group and optionally neutral units). Conditioning agents are known by the one skilled in the art. Examples of typical conditioning agents include (INCI names):
Polyquaternium-1; Polyquaternium-2; Polyquaternium-4; Polyquaternium-5; Polyquaternium-6; Polyquaternium-7; Polyquaternium-8; Polyquaternium-9; Polyquaternium-10; Polyquaternium-11; Polyquaternium-12; Polyquaternium-13; Polyquaternium-14; Polyquaternium-15; Polyquaternium-16; Polyquaternium-17; Polyquaternium-18; Polyquaternium-19; Polyquaternium-20; Polyquaternium-22; Polyquaternium-24; Polyquaternium-27; Polyquaternium-28; Polyquaternium-29; Polyquaternium-30; Polyquaternium-31; Polyquaternium-32; Polyquaternium-33; Polyquaternium-34 Polyquaternium-35; Polyquaternium-36; Polyquaternium-37; Polyquaternium-39; Polyquaternium-43; Polyquaternium-44; Polyquaternium-45; Polyquaternium-46; Polyquaternium-47; Polyquaternium-48; Polyquaternium-49; Polyquaternium-50; Polyquaternium-52; Polyquaternium-53; Polyquaternium-54; Polyquaternium-55; Polyquaternium-56; Polyquaternium-57; Polyquaternium-58; Polyquaternium-59; Polyquaternium-60; Polyquaternium-63; Polyquaternium-64; Polyquaternium-65; Polyquaternium-66; Polyquaternium-67; Polyquaternium-70; Polyquaternium-85; Polyquaternium-86; Polybeta-Alanine; Polyepsilon-Lysine; Polylysine; PEG-8/SMDI Copolymer; PPG-12/SMDI Copolymer; PPG-51/SMDI Copolymer; PPG-7/Succinic Acid Copolymer; IPDI/PEG-15 Cocamine Copolymer; IPDI/PEG-15 Cocamine Copolymer Dimer Dilinoleate; IPDI/PEG-15 Soyamine Copolymer; IPDI/PEG-15 Soyamine Oxide Copolymer; IPDI/PEG-15 Soyethonium Ethosulfate Copolymer; Polyquaternium-4/Hydroxypropyl Starch Copolymer; Cassia Hydroxypropyltrimonium Chloride; Chitosan Hydroxypropyltrimonium Chloride; Dextran Hydroxypropyltrimonium Chloride; Galactoarabinan Hydroxypropyltrimonium Chloride; Ginseng Hydroxypropyltrimonium Chloride; Guar Hydroxypropyltrimonium Chloride; Hydroxypropyl Guar Hydroxypropyltrimonium Chloride; Locust Bean Hydroxypropyltrimonium Chloride; Starch Hydroxypropyltrimonium Chlorid; Hydroxypropyltrimonium Hydrolyzed Wheat Starch; Hydroxypropyltrimonium Hydrolyzed Corn Starch; Hydroxypropyl Oxidized Starch PG-Trimonium Chloride; Tamarindus Indica Hydroxypropyltrimonium Chloride; Polyacrylamidopropyltrimonium Chloride; Polymethacrylamidopropyltrimonium Chloride; Polymethacrylamidopropyltrimonium Methosulfate; Propyltrimoniumchloride Methacrylamide/Dimethylacrylamide Copolymer; Acrylamide/Ethalkonium Chloride Acrylate Copolymer; Acrylamide/Ethyltrimonium Chloride Acrylate/Ethalkonium Chloride Acrylate Copolymer; Acrylates/Carbamate Copolymer; Adipic Acid/Methyl DEA Crosspolymer; Diethylene Glycol/DMAP Acrylamide/PEG-180/HDI Copolymer; Dihydroxyethyl Tallowamine/IPDI Copolymer; Dimethylamine/Ethylenediamine/Epichlorohydrin Copolymer; HEMA Glucoside/Ethylmethacrylate Trimonium Chloride Copolymer; Hydrolyzed Wheat Protein/PEG-20 Acetate Copolymer; Hydrolyzed Wheat Protein/PVP Crosspolymer; Ethyltrimonium Chloride Methacrylate/Hydroxyethylacrylamide Copolymer.

According to any one of the invention embodiments, the amount of conditioning agent in the composition can be in the range from 0.01 to 15 wt.%, for example from 0.05 to 10 wt.%, from 0.1 to 10 wt.%, from 0.1 to 5 wt.% based on the total weight of the composition.

### Additional surfactant

The composition of the present invention may further comprise one or more additional anionic surfactants. In some embodiments, said additional anionic surfactants may be selected from salts of alkyl sulfates, of alkylamide sulfates, of alkyl ether sulfates, of alkylamido ether sulfates, of alkylaryl ether sulfates, of monoglyceride sulfates and amino acid-based surfactant. Typical examples of such surfactants include sodium lauryl sulfate (SLS), sodium laureth sulfate (SLES), ammonium lauryl sulfate (ALS) or ammonium laureth sulfate (ALES). In some embodiments, the anionic surfactant, used either alone or as part of the nonionic/anionic surfactant mixture, can comprise, consist of, or consist essentially of a compound selected from the group consisting of an ammonium, alkali or earth alkali salt of: a sulfonate, a sulfosuccinate, a carboxylate, a sarcosinate, an isethionate, a sulfoacetate; and combinations thereof. In some preferred embodiments, the anionic surfactants used in the present invention include amino acid-based surfactants, such as N-acyl acidic amino acid and salts thereof, for example, N-acyl glutamic acid, N-acyl aspartic acid, their salts (sodium, potassium, ammonium or triethanolamine (TEA) salts or their mixtures.

The composition of the present invention may further comprise one or more amphoteric surfactants, which can comprise, consist of, or consist essentially of a compound selected from the group consisting of coco amido propyl betaine, cocoamido hydroxyl sultaine, cocamphoacetate, sodium methyl cocoyl taurate, and combinations thereof.

Nonionic surfactants suitable being used in the present invention, can comprise, consist of, or consist essentially of a compound selected from the group consisting of an alkyl polyglucoside, cocoamide monoethanolamine, cocoamide diethanolamine, a glycerol alkyl ester, polyethylene glycol, and combinations thereof. Preferably, the nonionic surfactant is alkyl polyglucoside for the present invention.

Suitable additional surfactant components for use in the personal care composition herein include those that are known for use in hair care or other personal care compositions.

According to any one of the invention embodiments, additional surfactants may be present in an amount ranging from 0.1 to 70 wt.% relative to the total weight of the composition, in particular, e.g. from 1 to 50 wt.%, 1 to 30 wt.% , still in particular from 5 to 20 wt.%, more in particular, from 10 to 15 wt.%.

For the avoidance of any doubt the amounts of surfactant refer to the actual amount of active surfactant compound present in the composition. In other words, it does not include the residue which may be present as an impurity in a commercially available surfactant mixture.

### Additional benefit agent

The compositions of the present invention may further comprise one or more benefit agents that can provide a positive and/or beneficial effect to the substrate being cleaned, e.g. to the hair and skin. The skilled person is able to select according to general knowledge in the art of formulating personal care compositions such as shampoos, shower gels and liquid hand soaps, and the vast literature there-related, appropriate such optional ingredients for application purposes. In one embodiment, the composition of the present invention further comprises one or more benefit agents, such as emollients, moisturizers, conditioners, skin conditioners, or hair conditioners such as silicones such as volatile silicones, gums or oils, or non-amino silicones and mixtures thereof, mineral oils, esters, such as butyl myristate, cetyl palmitate, decyloleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl myristate, isopropyl palmitate, isopropyl stearate, propylene glycol monolaurate, propylene glycol ricinoleate, propylene glycol stearate, and propylene glycol isostearate, animal fats, including acetylated lanolin alcohols, lanolin, lard, mink oil and tallow, and fatty acids and alcohols, including behenic acid, palmitic acid, stearic acid, behenyl alcohol, cetyl alcohol, eicosanyl alcohol and isocetyl alcohol; vitamins or their derivatives, such as vitamin B complex, including thiamine, nicotinic acid, biotin, pantothenic acid, choline, riboflavin, vitamin B6, vitamin B12, pyridoxine, inositol, carnitine, vitamins A,C,D,E,K and their derivatives, such as vitamin A palmitate, and pro-vitamins, e.g., panthenol (pro vitamin B5), panthenol triacetate and mixtures thereof; antioxidants; free-radical scavengers; abrasives, natural or synthetic; dyes; hair coloring agents; bleaching agents; hair bleaching agents; UV absorbers, such as benzophenone, bornelone, PABA (Para Amino Benzoic Acid), butyl PABA, cinnamidopropyl trimethyl ammonium chloride, disodium distyrylbiphenyl disulfonate, potassium methoxycinnamate; anti-UV agents, such as butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, octyl dimethyl PABA (padimate O), red petrolatum; antimicrobial agents; antibacterial agents, such as bacitracin, erythromycin, triclosan, neomycin, tetracycline, chlortetracycline, benzethonium chloride, phenol, parachlorometa xylenol (PCMX), triclocarban (TCC), chlorhexidine gluconate (CHG), zinc pyrithione, selenium sulfide; antifungal agents; melanin regulators; tanning accelerators; depigmenting agents, such as retinoids such as retinol, kojic acid and its derivatives such as, for example, kojic dipalmitate, hydroquinone and its derivatives such as arbutin, transexamic acid, vitamins such as niacin, vitamin C, azelaic acid, placertia, licorice, extracts such as chamomile and green tea, where retinol, kojic acid, and hydroquinone are preferred; skin lightening agents such as hydroquinone, catechol, ascorbic acid; skin coloring agents, such as dihydroxyacetone; liporegulators; weight-reduction agents; anti-acne agents; anti-seborrhoeic agents; anti-ageing agents; anti-wrinkle agents; keratolytic agents; anti-inflammatory agents; anti-acne agents, such as tretinoin, isotretinoin, motretinide, adapalene, tazarotene, azelaic acid, retinol, salicylic acid, benzoyl peroxide, resorcinol, antibiotics such as tetracycline and isomers thereof, erythromycin, anti-inflammatory agents such as ibuprofen, naproxen, hetprofen, botanical extracts such as alnus, arnica, artemisia capillaris, asiasarum root, calendula, chamomile, nidium, comfrey, fennel, galla rhois, hawthorn, houttuynia, hypericum, jujube, kiwi, licorice, magnolia, olive, peppermint, philodendron, salvia, sasa albomarginata, imidazoles such as ketoconazole and elubiol; refreshing agents; cicatrizing agents; vascularprotection agents; agents for the reduction of dandruff (Anti-dandruff agent), seborrheic dermatitis, or psoriasis, such as pyrithione salts, being formed from heavy metals such as zinc, tin, cadmium, magnesium aluminum, sodium and zirconium, like zinc pyrithione, shale oil and derivatives thereof such as sulfonated shale oil, selenium sulfide, sulfur, salicylic acid, coal tar, povidone-iodine, imidazoles such as ketoconazole, dichlorophenyl imidazolodioxalan, clotrimazole, itraconazole, miconazole, climbazole, tioconazole, sulconazole, butoconazole, fluconazole, miconazolenitrite and any possible stereo isomers and derivatives thereof such as anthralin, piroctone olamine (Octopirox), selenium sulfide, ciclopirox olamine, anti-psoriasis agents such as vitamin D analogs, e.g. calcipotriol, calcitriol, and tacaleitrol, vitamin A analogs such as esters of vitamin A, including vitamin A palmitate, retinoids, retinols, and retinoic acid, corticosteroids such as hydrocortisone, clobetasone, butyrate, clobetasol propionate; antiperspirants or deodorants, such as aluminum chlorohydrates, aluminum zirconium chlorohydrates; immunomodulators; nourishing agents; depilating agents, such as calcium thioglycolate, magnesium thioglycolate, potassium thioglycolate, strontium thioglycolate; agents for combating hair loss; reducing agents for permanent-waving; reflectants, such as mica, alumina, calcium silicate, glycol dioleate, glycol distearate, silica, sodium magnesium fluorosilicate; essential oils and fragrances.

According to some invention embodiments, the composition of the present invention may further comprise polymeric or non-polymeric thickener to make the composition have satisfactory viscosity for use purpose. The composition may comprise less than 10 wt.% of an additional thickener. Suitable classes of thickeners for the present composition include, but are not limited to sodium chloride, potassium chloride, ammonium chloride, sodium sulfate, fatty acid alkylolamides, cellulose derivatives, carboxylic acid polymers, crosslinked polyacrylate polymers, polyacrylamide polymers, polysaccharides, gums and mixtures thereof. Non-limiting examples of suitable thickening agents are described in the CTFA International Cosmetic Ingredient Dictionary, 10th Ed. (2004), pp. 2294 - 96.

According to any one of the invention embodiments, the composition further comprises a cosmetically acceptable carrier. In some embodiment, the carrier is an aqueous carrier. The amount and chemistry of the carrier is selected according to the compatibility with other components and others desired characteristic of the product. In some embodiments, the carrier is selected from the group consisting of water and water solutions of lower alkyl alcohols. In some embodiments, the carrier is a cosmetically acceptable aqueous carrier and is present at a level of from about 20% to about 95%, or from about 60 % to about 85%.

According to any one of the invention embodiments, the personal care composition is used in a manner know in the art, for example, in the case of a cleanser or shampoo, by application of the cleanser or shampoo to the skin and/or hair and optionally rinsing the cleanser or shampoo off of the skin and/or hair with water. According to any one of the invention embodiments, the composition of the invention may have a pH comprised between 4 and 11, for instance between 4 and 6.

The personal care composition of the present invention can be prepared by mixing individual components using any conventional blending technique known in the prior art such as conventional stirring, shaking or tumbling. These components may be supplied as concentrated solutions which are diluted and/or and combined in appropriate ratios by the skilled person. The invention covers any concentrate to be used as component ingredient to prepare a composition of the invention, and especially to concentrates containing limited levels of water due to some reasons from a cost and environmental perspective.

### Examples:

### Materials

Cocamidopropyl Betaine: Dehyton^{®} PK45 from BASF
Disodium Cocoyl Glutamate: Plantapon^{®} ACG 50 from BASF
Sodium Cocoyl Isethionate: Jordapon^{®} CILA from BASF
Coco Glycoside: Plantacare^{®} 818UP from BASF
Sodium Laureth Sulfate: Texapon^{®} NSO from BASF
Sophorolipid: ACS-Sophor^{®} from Allied Carbon Solution
Polyquaternium-10: Ucare^{®} JR400 from Dow
Acrylamidopropyltrimonium Chloride/Acrylamide Copolymer: Salcare^{®} SC60 from BASF
PEG/PPG-120/10 Trimethylolpropane Trioleate (and) Laureth-2: Arylpon^{®} TT from BASF
Hexamidine Diisethionate: Elestab^{®} HP100 from BASF
Quinine Sulfate: BR, SCRC^{®} from Sinoreagent

### Formulation protocol

1. Cationic polymer (Polyquaternium-10, Guar Hydroxypropyltrimonium Chloride and Acrylamidopropyltrimonium Chloride/Acrylamide Copolymer) was added in water (60 mL) under stirring at 200 rpm until the cationic polymer was homogeneous dispersed and fully swollen in water.
2. Surfactants were added in the solution containing polymer (obtained in step 1) in turn while under stirring at 200 rpm. Sodium Cocoyl Isethionate need to be heated to 70 degree C to completely dissolved in the solution.
3. Hydrophile cationic or pseudo-cationic active ingredient was dissolved in water (5 mL) under stirring at 200 rpm, and then added into the solution obtained in step 2 under stirring at 200 rpm until a homogeneous system.
4. Other ingredients including thickener (PEG/PPG-120/10 Trimethylolpropane Trioleate (and) Laureth-2), preservatives (sodium benzoate) and sodium chloride were added into the solution obtained in step 3 in turn under stirring at 200 rpm.
5. Water was added to the solution obtained in step 4 to 100g and the pH of the formulation solution was adjusted to 4.5-5.5 with citric acid solution or triethanolamine.

**Formulation details**

| Ingredients | Formulations (wt.% active content ingredients) | | | | | | | | | |
|---|---|---|---|---|---|---|---|---|---|---|
| | Ex. 1 | Comp Ex.1 | Ex. 2 | Comp Ex.2 | Ex. 3 | Comp Ex. 3A | Comp Ex. 3B | Ex. 4 | Comp Ex. 4A | Comp Ex. 4B |
| Cocamidopropyl Betaine | 3.8 | 4.0 | 3.8 | 4.0 | 4.86 | 4.86 | 4.86 | 4.86 | 4 | 4.86 |
| Disodium Cocoyl Glutamate | - | | | | 2.4 | 2.4 | 2.4 | 2.4 | - | 2.4 |
| Sodium Cocoyl Isethionate | | | | | 4 | 4 | 4 | 4 | - | 4 |
| Coco Glycoside | - | | | | - | 5 | - | - | - | 5 |
| Sodium Laureth Sulfate | 9.45 | 10 | 9.45 | 10 | - | - | 5 | - | 10 | - |
| Sophorolipid | 0.75 | - | 0.75 | - | 5 | - | - | 5 | - | - |
| Polyquaternium-10 | - | - | 0.3 | 0.3 | 0.3 | 0.3 | 0.3 | 0.3 | 0.3 | 0.3 |
| Acrylamidopropyltrimonium Chloride/Acrylamide Copolymer | 0.3 | 0.3 | - | - | - | - | - | - | - | - |
| PEG/PPG-120/10 Trimethylolpropane Trioleate (and) Laureth-2 | - | - | - | - | 0.65 | 0.65 | 0.65 | 0.65 | - | 0.65 |
| Sodium Chloride | 0.5 | 0.5 | 0.5 | 0.5 | - | - | - | - | 0.5 | - |
| Hexamidine Diisethionate | 0.1 | 0.1 | 0.1 | 0.1 | 0.1 | 0.1 | 0.1 | - | - | - |
| Quinine Sulfate | - | - | - | - | - | - | - | 0.3 | 0.3 | 0.3 |
| Sodium Benzoate | 0.5 | 0.5 | 0.5 | 0.5 | 0.5 | 0.5 | 0.5 | 0.5 | 0.5 | 0.5 |
| Water | To 100 | To 100 | To 100 | To 100 | To 100 | To 100 | To 100 | To 100 | To 100 | To 100 |

| | | | | | | | | | | |
|---|---|---|---|---|---|---|---|---|---|---|
| Ex. = Inventive Example; Comp. Ex. = Comparative Example; "-" = not contained | | | | | | | | | | |

### Evaluation method

A piece of wool fabric (5cm x 5cm, 0.6 g) was placed in the bottom of a 150 mL beaker, then 3g of the formulation with active ingredient was added onto the fabric and 12 g of D.I water was added to dilute. The diluted formulation was stirred above the fabric with a magnetic stirring bar for 1h at 100 rpm to provide sufficient time for coacervation deposition. The wool fabric was then taken out from the beaker and was stirred in 500 g of purified water for 15 seconds to remove loosely attached coacervate deposition. The wool fabric was then moved into a centrifuge and submerged with 30g of MeOH overnight until attached coacervates and active ingredient is fully dissolved. The supernatant was collected by centrifugation at 3000 rpm for 10 mins after removing the wool fabric from the tube.

### Deposition measurement method

The collected supernatant was tested in MeOH by using ultraviolet spectrophotometric method (Perkin Elmer 365) at specific wavelength (265nm for hexamidine and 280 nm for quinine). UV absorption value deducting blank control which was measured using the formulation without active ingredients, is then used to calculate the active ingredient's amount in MeOH based on standard curve. The deposition rate is determined by dividing the initial active ingredient's amount added into the formulation.

### Deposition Rates of the hydrophile cationic or pseudo-cationic active ingredients

| | Formulations | | | | | | | | | |
|---|---|---|---|---|---|---|---|---|---|---|
| | Ex. 1 | Comp Ex.1 | Ex. 2 | Comp Ex.2 | Ex. 3 | Comp Ex. 3A | Comp Ex. 3B | Ex. 4 | Comp Ex. 4A | Comp Ex. 4B |
| Rate (%) | 0.75 | 0.47 | 5.42 | 1.18 | 12.80 | 4.45 | 7.00 | 7.78 | 5.14 | 6.06 |

| | | | | | | | | | | |
|---|---|---|---|---|---|---|---|---|---|---|
| Ex. = Example; Comp Ex.= Comparative Example | | | | | | | | | | |

The deposition test results demonstrate that the Inventive Examples comprising sophorolipid surfactants lead to substantial and surprising boost in deposition of hydrophile cationic or pseudo-cationic active ingredients in various personal care formulations. Due to the presence of sophorolipid surfactant, the Inventive Example 1 (Ex. 1) has higher deposition rate of the active ingredient (Hexamidine Diisethionate) than Comparative Example 1 (Comp Ex. 1); the Inventive Example 2 (Ex. 2) has much higher deposition rate of the active ingredient (Hexamidine Diisethionate) than Comparative Example 2 (Comp Ex. 2); The Inventive Example 3 (Ex. 3) has improved deposition effect on the active ingredient (Hexamidine Diisethionate) than Comparative Examples 3A & 3B (Comp Ex. 3A & 3B); and regarding the active ingredient quinine sulfate, the Example 4 containing sophorolipid surfactant, shows enhanced deposition rate than the Comparative Examples 4A & 4B (Comp Ex. 4A & 4B).

## Claims

1. A personal care composition comprising at least:
a) a glycolipid biosurfactant, and b) a hydrophile cationic or pseudo-cationic active compound; wherein the glycolipid is sophorolipid, and wherein the hydrophile cationic or pseudo-cationic active compound comprises one or more amidine compounds or salts thereof and/or one or more alkaloid compounds selected from the group consisting of quinine and its salts.

2. The composition according to claim 1, wherein the sophorolipid biosurfactant is a mixture of acidic form and lactone form; preferably 10 to 90 wt.%, more preferably 20 to 60 wt.%, still more preferably 25 wt.% to 40 wt.% of the sophorolipid being in acidic form and the remainder of the sophorolipid being in the lactone form.

3. The composition of any one of the preceding claims, wherein the sophorolipid is present in an amount ranging from 0.1 % to 30 wt. % of sophorolipid surfactant relative to the total weight of the composition, preferably from 0.2 % to 20 wt.%, more preferably from 0.2 % to 15 wt.%, still more preferably from 0.5 % to 10 wt.%..

4. The composition of any one of the preceding claims, wherein the hydrophile cationic or pseudo-cationic active compound comprises amidine compound, or its salt, in particular hexamidine compound, its salt or its isomers or tautomers; preferably the amidine compound is present in an amount ranging from 0.0001% to 10%, more preferably from 0.001% to 5% and more preferably still from 0.01% to 1%, and most preferably from 0.02% to 0.5%

5. The composition according to claims 1 to 3, wherein the hydrophile cationic or pseudo-cationic active compound comprises quinine or its salts; preferably the alkaloid compound is present in an amount ranging from 0.001% to 20%, more preferably from 0.01% to 10%, and more preferably still from 0.1% to 5% by weight of the composition.

6. The composition of any one of the preceding claims, further comprises at least one conditioning agent, for example a cationic or ampholytic conditioning agent; preferably the composition comprises a conditioning agent selected from the group consisting of cationic polysaccharides.

7. The composition of any one of the preceding claims, further comprises one or more anionic surfactants, nonionic surfactants and/or amphoteric surfactants; in particular, the surfactants comprise amino acid-based surfactant, such as N-acyl acidic amino acid and salts thereof, and/or alkyl polyglucoside.

8. The composition of any one of the preceding claims, further comprising one or more additional benefit agent.

9. The composition according to any one of the preceding claims, being a rinse-off product.

10. A method of enhancing or facilitating a deposition of hydrophile cationic or pseudo-cationic active compound onto keratinous materials, comprising at least one step of applying to the keratinous materials an effective amount of the composition as defined in any one of the preceding claims.

11. Use of the composition as defined in any one of claims 1 to 9 for enhancing or facilitating a deposition of hydrophile cationic or pseudo-cationic active compound onto keratinous materials.

12. Use of the composition as defined in any one of claims 1 to 9 for simultaneously caring for and washing keratinous materials, such as hair and skin.

## Patentansprüche

1. Eine Körperpflegezusammensetzung, umfassend mindestens:
a) ein Glykolipid-Biotensid und b) eine hydrophile kationische oder pseudo-kationische Wirkverbindung; wobei das Glykolipid ein Sophorolipid ist und wobei die hydrophile kationische oder pseudo-kationische Wirkverbindung eine oder mehrere Amidinverbindungen oder deren Salze und/oder eine oder mehrere Alkaloidverbindungen umfasst, die aus der Gruppe bestehend aus Chinin und seinen Salzen ausgewählt sind.

2. Die Zusammensetzung gemäß Anspruch 1, wobei das Sophorolipid-Biotensid ein Gemisch aus saurer Form und Lactonform ist; vorzugsweise 10 bis 90 Gew.-%, bevorzugter 20 bis 60 Gew.-%, noch bevorzugter 25 Gew.-% bis 40 Gew.-% des Sophorolipids in saurer Form vorliegen und der Rest des Sophorolipids in der Lactonform vorliegt.

3. Die Zusammensetzung nach einem der vorhergehenden Ansprüche, wobei das Sophorolipid in einer Menge im Bereich von 0,1 % bis 30 Gew.-% Sophorolipid-Tensid bezogen auf das Gesamtgewicht der Zusammensetzung vorhanden ist, vorzugsweise von 0,2 % bis 20 Gew.-%, bevorzugter von 0,2 % bis 15 Gew.-%, noch bevorzugter von 0,5 % bis 10 Gew.-%..

4. Die Zusammensetzung nach einem der vorhergehenden Ansprüche, wobei die hydrophile kationische oder pseudo- kationische Wirkverbindung eine Amidinverbindung oder ihr Salz umfasst, insbesondere eine Hexamidinverbindung, ihr Salz oder ihre Isomere oder Tautomere; vorzugsweise ist die Amidinverbindung in einer Menge im Bereich von 0,0001 % bis 10 %, bevorzugter von 0,001 % bis 5 % und noch bevorzugter von 0,01 % bis 1 % und am bevorzugtesten von 0,02 % bis 0,5 % vorhanden.

5. Die Zusammensetzung gemäß den Ansprüchen 1 bis 3, wobei die hydrophile kationische oder pseudo-kationische Wirkverbindung Chinin oder seine Salze umfasst; vorzugsweise ist die Alkaloidverbindung in einer Menge im Bereich von 0,001 % bis 20 %, bevorzugter von 0,01 % bis 10 % und noch bevorzugter von 0,1 % bis 5 % bezogen auf das Gewicht der Zusammensetzung vorhanden.

6. Die Zusammensetzung nach einem der vorhergehenden Ansprüche, die ferner mindestens ein Konditioniermittel umfasst, beispielsweise ein kationisches oder ampholytisches Konditioniermittel; vorzugsweise umfasst die Zusammensetzung ein Konditioniermittel, das aus der Gruppe bestehend aus kationischen Polysacchariden ausgewählt ist.

7. Die Zusammensetzung nach einem der vorhergehenden Ansprüche, die ferner ein oder mehrere anionische Tenside, nichtionische Tenside und/oder amphotere Tenside umfasst; insbesondere umfassen die Tenside ein auf Aminosäuren basierendes Tensid, wie N-Acyl-saure Aminosäure und deren Salze, und/oder Alkylpolyglucosid.

8. Die Zusammensetzung nach einem der vorhergehenden Ansprüche, die ferner ein oder mehrere zusätzliche Nutzungsmittel umfasst.

9. Die Zusammensetzung nach einem der vorhergehenden Ansprüche, die ein Ausspülprodukt ist.

10. Ein Verfahren zur Verbesserung oder Erleichterung einer Ablagerung einer hydrophilen kationischen oder pseudo-kationischen Wirkverbindung auf keratinösen Materialien, umfassend mindestens einen Schritt des Auftragens einer wirksamen Menge der Zusammensetzung gemäß einem der vorhergehenden Ansprüche auf die keratinösen Materialien.

11. Verwendung der Zusammensetzung gemäß einem der Ansprüche 1 bis 9 zur Verbesserung oder Erleichterung einer Ablagerung einer hydrophilen kationischen oder pseudo-kationischen Wirkverbindung auf keratinösen Materialien.

12. Verwendung der Zusammensetzung gemäß einem der Ansprüche 1 bis 9 zur gleichzeitigen Pflege und Reinigung keratinöser Materialien, wie Haare und Haut.

## Revendications

1. Une composition de soin personnel comprenant au moins :
a) un biosurfactant glycolipidique, et b) un composé actif hydrophile cationique ou pseudo-cationique ; dans laquelle le glycolipide est un sophorolipide, et dans laquelle le composé actif hydrophile cationique ou pseudo-cationique comprend un ou plusieurs composés amidine ou leurs sels et/ou un ou plusieurs composés alcaloïdes choisis dans le groupe constitué par la quinine et ses sels.

2. La composition selon la revendication 1, dans laquelle le biosurfactant sophorolipide est un mélange de forme acide et de forme lactone ; de préférence 10 à 90 % en poids, plus préférablement 20 à 60 % en poids, encore plus préférablement 25 % à 40 % en poids du sophorolipide étant sous forme acide et le reste du sophorolipide étant sous forme lactone.

3. La composition selon l'une quelconque des revendications précédentes, dans laquelle le sophorolipide est présent en une quantité allant de 0,1 % à 30 % en poids de tensioactif sophorolipide par rapport au poids total de la composition, de préférence de 0,2 % à 20 % en poids, plus préférablement de 0,2 % à 15 % en poids, encore plus préférablement de 0,5 % à 10 % en poids.

4. La composition selon l'une quelconque des revendications précédentes, dans laquelle le composé actif hydrophile cationique ou pseudo-
cationique comprend un composé amidine, ou son sel, en particulier un composé hexamidine, son sel ou ses isomères ou tautomères ; de préférence le composé amidine est présent en une quantité allant de 0,0001 % à 10 %, plus préférablement de 0,001 % à 5 % et encore plus préférablement de 0,01 % à 1 %, et le plus préférablement de 0,02 % à 0,5 %

5. La composition selon les revendications 1 à 3, dans laquelle le composé actif hydrophile cationique ou pseudocationique comprend la quinine ou ses sels ; de préférence le composé alcaloïde est présent en une quantité allant de 0,001 % à 20 %, plus préférablement de 0,01 % à 10 %, et encore plus préférablement de 0,1 % à 5 % en poids de la composition.

6. La composition selon l'une quelconque des revendications précédentes, comprenant en outre au moins un agent conditionnant, par exemple un agent conditionant cationique ou ampholytique ; de préférence la composition comprend un agent conditionant choisi dans le groupe constitué par les polysaccharides cationiques.

7. La composition selon l'une quelconque des revendications précédentes, comprenant en outre un ou plusieurs tensioactifs anioniques, tensioactifs non ioniques et/ou tensioactifs amphotères ; en particulier, les tensioactifs comprennent un tensioactif à base d'acides aminés, tel qu'un N-acyl acide aminé acide et ses sels, et/ou un alkylpolyglucoside.

8. La composition selon l'une quelconque des revendications précédentes, comprenant en outre un ou plusieurs agents bénéfiques supplémentaires.

9. La composition selon l'une quelconque des revendications précédentes, étant un produit à rincer.

10. Un procédé pour améliorer ou faciliter le dépôt d'un composé actif hydrophile cationique ou pseudo-cationique sur des matières kératineuses, comprenant au moins une étape consistant à appliquer sur les matières kératineuses une quantité efficace de la composition telle que définie dans l'une quelconque des revendications précédentes.

11. Utilisation de la composition telle que définie dans l'une quelconque des revendications 1 à 9 pour améliorer ou faciliter le dépôt d'un composé actif hydrophile cationique ou pseudo-cationique sur des matières kératineuses.

12. Utilisation de la composition telle que définie dans l'une quelconque des revendications 1 à 9 pour prendre soin simultanément et laver des matières kératineuses, telles que les cheveux et la peau.
